(19) Europäisches Patentamt
European Patent Office
Office européen des brevets

(11) **EP 4 785 952 A1**

(12) **EUROPEAN PATENT APPLICATION**
published in accordance with Art. 153(4) EPC

(43) Date of publication:
**05.08.2026   Bulletin 2026/32**

(21) Application number: **24870921.4**

(22) Date of filing: **27.09.2024**

(51) International Patent Classification (IPC):
*A61K 39/395* (2006.01)    *A61K 38/17* (2006.01)
*A61P 37/06* (2006.01)

(52) Cooperative Patent Classification (CPC):
**A61K 38/17; A61K 39/395; A61P 37/06**

(86) International application number:
**PCT/CN2024/121600**

(87) International publication number:
**WO 2025/067383 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   28.09.2023   CN 202311272629

(71) Applicant: **RemeGen Co., Ltd.
Yantai, Shandong 264006 (CN)**

(72) Inventors:
• **FANG, Jianmin
  Yantai, Shandong 264006 (CN)**
• **WANG, Wenxiang
  Yantai, Shandong 264006 (CN)**
• **WU, Jingping
  Yantai, Shandong 264006 (CN)**

(74) Representative: **Cooley (UK) LLP
22 Bishopsgate
London EC2N 4BQ (GB)**

Remarks:
The complete document including Reference
Table(s) and the Sequence Listing(s) can be
downloaded from the EPO website

(54)   **METHOD FOR TREATING ANTIBODY-MEDIATED REJECTION USING TACI-FC FUSION PROTEIN**

(57)   The present invention relates to a drug, a dosage regimen, a dosing interval, and an administration mode for treating antibody-mediated rejection using an effective amount of drugs that target Blys and/or APRIL. The results show that the effective amount of provided drugs that target Blys and/or APRIL exhibit good safety and therapeutic effects in the prevention, treatment or mitigation of antibody-mediated rejection in patients who are to accept, are accepting or have accepted solid organ transplantation.

FIG. 1

**Description**

**Technical Field**

The present invention relates to a TACI-Fc fusion protein medicament for treating antibody-mediated rejection, a dosage regimen, a dosing interval, and an administration mode.

**Background Art**

Antibody-mediated rejection (ABMR), referred to as humoral rejection for short, is the most common cause of late allograft loss after organ transplantation and a major obstacle affecting long-term graft survival [Reference 1: Sellarés J, de Freitas DG, Mengel M, et al. Understanding the causes of kidney transplant failure: the dominant role of antibody-mediated rejection and nonadherence. Am J Transplant 2012; 12:388-399]. According to the Banff (2019) classification criteria, antibody-mediated rejection is mainly divided into four categories: Active ABMR (also referred to as Acute active ABMR), Chronic active ABMR, Chronic inactive ABMR, and C4d staining without evidence of rejection [Reference 2: Loupy A, et al. The Banff 2019 Kidney Meeting Report (I): Updates on and clarification of criteria for T cell- and antibody-mediated rejection. Am J Transplant. 2020 Sep;20(9):2318-2331.].

Currently, no therapeutic drug for antibody-mediated rejection has been approved for commercial marketing worldwide, and only some published randomized clinical trials have evaluated treatment regimens for antibody-mediated rejection in kidney transplant recipients [see https://clinicaltrials.gov/search?cond=ABMR, https://clinicaltrials.gov/search?cond=AMR]. However, most studies have small sample sizes and are unable to detect differences between treatment regimens, which results in significant heterogeneity in the treatment of antibody-mediated rejection. Currently, patients with active antibody-mediated rejection are commonly treated clinically with combined antibody removal (i.e., removal of antibodies already present or newly generated in the patient), glucocorticoids, intravenous immunoglobulin, anti-CD20 antibodies, proteasome inhibitors and/or complement blockade, but because chronic active antibody-mediated rejection can cause irreversible damage to graft tissue, resulting in significantly reduced graft survival, no treatment method has been proven to be effective for the treatment of chronic active antibody-mediated rejection [Reference 3: Rodriguez-Ramirez S, Al Jurdi A, Konvalinka A, Riella LV. Antibody-mediated rejection: prevention, monitoring and treatment dilemmas. Curr Opin Organ Transplant. 2022 Oct 1;27(5):405-414.]. In addition, although two studies have indicated that plasma exchange in the treatment of antibody-mediated rejection can improve allograft survival [Reference 4: Bonomini V, Vangelista A, Frascà GM, et al. Effects of plasmapheresis in renal transplant rejection. A controlled study. Trans Am Soc Artif Intern Organs 1985; 31:698-703.], no significant benefit has been shown in some other plasma exchange trials [Reference 5: Allen NH, Dyer P, Geoghegan T. Plasma exchange in acute renal allograft rejection. A controlled trial. Transplantation 1983; 35:425-428.]. Regarding anti-CD20 treatment, a randomized clinical trial has shown that the addition of a single dose of rituximab to glucocorticoid, plasma exchange, and immunoglobulin treatment also did not improve allograft function or survival [Reference 6: Sautenet B, Blancho G, Büchler M, et al. One-year results of the effects of rituximab on acute antibody-mediated rejection in renal transplantation: RITUX ERAH, a multicenter double-blind randomized placebo-controlled trial. Transplantation 2016; 100:391-399.]. In addition, there are findings indicating that the combined application of immunoglobulin and rituximab may shorten the half-life of anti-CD20 monoclonal antibody, accelerate B cell recovery, and thus affect the efficacy of anti-CD20 [Reference 7: Laws LH, Parker CE, Cherala G, et al. Inflammation causes resistance to anti-CD20-mediated B cell depletion. Am J Transplant 2016; 16:3139-3149.]. Therefore, developing and establishing a treatment regimen to improve the long-term prognosis of antibody-mediated rejection in post-transplantation patients remains an urgent unmet need.

Currently, organ transplantation remains the most effective form of treatment for patients with end-stage renal disease and the like, but approximately 30% of patients awaiting transplantation are sensitized due to history of blood transfusion, pregnancy, or prior transplantation, leading to pre-existing donor-specific antibodies (DSA), wherein the major component of DSA is anti-human leucocyte antigen (HLA) antibody. According to the preoperative panel reactive antibodies (PRA) detection results, PRA>10% is generally defined as sensitized, and PRA>50% is defined as highly sensitized [Reference 8: Jordan SC, Tyan D, Stablein D, et al. Evaluation of intravenous immunoglobulin as an agent to lower allosensitization and improve transplantation in highly sensitized adult patients with end-stage renal disease: report of the NIH IG02 trial[J]. J Am Soc Nephrol 2004, 15(12): 3256-3262.; Reference 9: Vieira CA, Agarwal A, Book BK, et al. Rituximab for reduction of anti-HLA antibodies in patients awaiting renal transplantation: 1. Safety, pharmacodynamics, and pharmacokinetics[J]. Transplantation 2004, 77(4): 542-548.]. Sensitized patients typically have difficulty obtaining crossmatch-compatible donor kidneys, and the risk of antibody-mediated rejection after transplantation is increased. Therefore, HLA antibody desensitization treatment is required before renal transplantation to deplete circulating DSA of highly sensitized patients through physical or pharmacotherapeutic means and to specifically inhibit T and B cell activation and block DSA synthesis, so as to prevent hyperacute rejection and antibody-mediated rejection from occurring, thereby enabling highly sensitized patients to successfully undergo transplantation [Reference 10: Wang Zhiyong, Zhang Geng, Yuan Jianlin. Research progress in preoperative desensitization treatment for pre-sensitized patients with kidney disease before transplantation [J]. Chinese Journal of Organ Transplantation 2017, 38(12): 761-764.].

Povetacicept (ALPN-303) is a Fc fusion protein of an engineered TACI domain, which, as a dual BAFF/APRIL antagonist, is being explored for the treatment of lupus nephritis (Phase I/II), membranous nephropathy (Phase I/II), IgA nephropathy (Phase I/II), immune thrombocytopenia (Phase I/II), cold agglutinin disease (Phase I/II), warm antibody autoimmune hemolytic anemia (Phase I/II), systemic lupus erythematosus (Phase I), autoimmune cytopenia (Phase I), myasthenia gravis (preclinical) and other indications, and is currently in different clinical stages.

Currently, Alpine Immune Sciences has disclosed three povetacicept clinical trial information, wherein RUBY 1 (NCT05034484) is a Phase I safety study in healthy volunteers, demonstrating that povetacicept exhibits acceptable safety and tolerability, as well as expected pharmacodynamic (PD) effects on circulating immunoglobulins (Ig). RUBY 3 (NCT05732402) is an open-label Phase 1b/2a study of povetacicept for the treatment of Autoimmune Kidney Diseases, and the preliminary study results showed that povetacicept 80 mg SC Q4W, after multiple dosing in patients with IgA nephropathy, demonstrated good tolerability and is expected to reduce urinary protein-to-creatinine ratio (UPCR) and Gd-IgA1. RUBY 4 (NCT05757570) is an ongoing open-label Phase 1b study of povetacicept for the treatment of autoimmune cytopenias. In addition, povetacicept is intended for the treatment of inflammatory diseases involving BAFF and/or APRIL, such as systemic lupus erythematosus, and is currently undergoing a clinical trial conducted in China (registration number CTR20234038).

Atacicept is a recombinant fusion protein comprising the extracellular ligand-binding portion of the TACI receptor and the Fc portion of human IgG, and is capable of binding to BLyS and APRIL. The drug is being explored for the treatment of indications such as IgA nephropathy (Phase III), lupus nephritis (Phase III), systemic lupus erythematosus (Phase II/III), autoimmune diseases, and is currently at different clinical stages.

The Phase 2b ORIGIN trial (NCT04716231) of atacicept for the treatment of IgA nephropathy obtained positive results, with atacicept treatment achieving the primary endpoint and the key secondary endpoint. In addition, the safety profiles of the atacicept and placebo treatment groups were comparable. A Phase 3 clinical trial (NCT04716231) of atacicept for the treatment of IgA nephropathy is recruiting, with the treatment group receiving atacicept at a dose of 150 mg per administration, subcutaneously, once a week. In addition, a Phase IIb/III clinical trial (registration number: CTR20242150) evaluating the efficacy and safety of atacicept in subjects with IgA nephropathy (IgAN) is currently being conducted in China. Results from a Phase III clinical trial (NCT00624338) of atacicept for the efficacy and safety in prevention of moderate to severe systemic lupus erythematosus (SLE) showed that, compared with the placebo group, only the 150 mg dose atacicept group demonstrated efficacy (43% and 60%; OR 0.49 (0.26, 0.92), p=0.027), but due to two fatalities resulting from pneumonia complicated by pulmonary hemorrhage, the trial was terminated early for the atacicept 150 mg group. Three completed Phase II clinical trials of atacicept for the treatment of rheumatoid arthritis, NCT00595413, NCT00430495, and NCT00664521, were all terminated because the primary endpoints were not met.

Telitacicept is a first-in-class recombinant TACI-Fc fusion protein for B cell-related autoimmune diseases, which is capable of targeting and neutralizing two key cell signaling molecules, B-lymphocyte stimulator (BLyS) and a proliferation-inducing ligand (APRIL), in the B cell pathway. It is an antibody-like fusion protein composed of truncated TACI and an immunoglobulin Fc region optimized to exhibit reduced antibody-dependent cellular cytotoxicity (ADCC) and complement-dependent cytotoxicity (CDC) activities for the treatment of human autoimmune system diseases, has excellent biological activity and safety, and has been approved for marketing in China for the treatment of systemic lupus erythematosus.

**Summary of the Invention**

The present invention has been surprisingly discovered, during the course of treating real-world cases, that the TACI-Fc fusion protein has excellent biological activity and safety in preventing and treating patients with antibody-mediated rejection, producing significant therapeutic effects.

For this purpose, the present invention provides a method for preventing, treating or alleviating antibody-mediated rejection in patients who will receive, are receiving or have received solid organ transplantation, the method comprising administering to a patient having the antibody-mediated rejection a therapeutically effective amount of a drug targeting Blys and/or APRIL.

The present invention further provides a use of a drug targeting Blys and/or APRIL in the preparation of a medicament for preventing, treating or alleviating antibody-mediated rejection in a patient.

The present invention further provides the use of TACI-Fc fusion protein in the preparation of a drug for preventing, treating, or alleviating antibody-mediated rejection in a patient.

The present invention further provides the use of telitacicept in the preparation of a drug for preventing, treating or alleviating antibody-mediated rejection in a patient.

Further, the above-mentioned drug targeting Blys and/or APRIL is a TACI-Fc fusion protein.

Further, the TACI-Fc fusion protein according to any one of the above comprises:

(i) a TACI extracellular region or a fragment thereof that binds Blys and/or APRIL; and
(ii) a human immunoglobulin constant region fragment.

Further, the TACI extracellular region or a fragment thereof that binds Blys and/or APRIL comprises the amino acid sequence set forth in SEQ ID NO: 1 or SEQ ID NO:2 or SEQ ID NO:3.

SEQ ID NO:1

```
SRVDQEERFP QGLWTGVAMR SCPEEQYWDP LLGTCMSCKT ICNHQSQRTC AAFCRSLSCR    60
KEQGKFYDHL LRDCISCASI CGQHPKQCAY FCENKLRSPV NLPPEL                   106
```

SEQ ID NO:2

```
AMRSCPEEQY WDPLLGTCMS CKTICNHQSQ RTCAAFCRSL SCRKEQGKFY DHLLRDCISC    60
ASICGQHPKQ CAYFCENKLR S                                             81
```

SEQ ID NO:3
SLSCRKEQGE YYDHLLRDCI SCASICGQHP KQCADFCENK LRS 43

Further, the amino acid sequence of the TACI extracellular region or a fragment thereof that binds Blys and/or APRIL is as set forth in SEQ ID NO: 1.
Further, the human immunoglobulin is IgG1.
Further, the human immunoglobulin constant region fragment comprises the amino acid sequence of SEQ ID NO:4.
Further, the human immunoglobulin constant region fragment comprises an amino acid sequence having at least 90%, at least 91%, at least 92%, at least 93%, at least 94%, at least 95%, at least 96%, at least 97%, at least 98%, or at least 99% identity to SEQ ID NO:4.

SEQ ID NO:4

```
DKPHTCPLCP APELLGGPSV FLFPPKPKDT LMISRTPEVT CVVVDVSHED PEVKFNWYVD    60
GVEVHNAKTK PREEQYNSTY RVVSVLTVLH QDWLNGKEYK CKVSNKALPA PIEKTISKAK    120
GQPREPQVYT LPPSRDELTK NQVSLTCLVK GFYPSDIAVE WESNGQPENN YKATPPVLDS    180
DGSFFLYSKL TVDKSRWQQG NVFSCSVMHE ALHNHYTQKS LSLSPGK                 227
```

Further, the amino acid sequence of the human immunoglobulin constant region fragment is as set forth in SEQ ID NO:4.
Further, the human immunoglobulin constant region fragment comprises a modification of an amino acid on one or more positions corresponding to positions 3, 8, 14, 15, 17, 110, 111 or 173 of SEQ ID NO:4.
Further, the human immunoglobulin constant region fragment comprises amino acid modifications at 1, 2, 3, 4, 5, 6, 7, 8, or more positions as compared to SEQ ID NO:4.
Further, the modification is a substitution, deletion, or insertion of amino acids.
Further, the substitution comprises one or more of P3T, L8P, L14A, L15E, G17A, A110S, P111S, and A173T.
Further, the insertion is the insertion of 1, 2, 3, 4, 5, 6, 7, 8 or more amino acids at the N-terminus of the human immunoglobulin constant region fragment.
Still further, the insertion is the insertion of 5 amino acids at the N-terminus of the human immunoglobulin constant region fragment.
Still further, the insertion is the insertion of the 5 amino acids EPKSS at the N-terminus of the human immunoglobulin constant region fragment.
Further, the human immunoglobulin constant region fragment comprises the amino acid sequence of SEQ ID NO:5.

SEQ ID NO:5

```
DKTHTCPPCP APEAEGAPSV FLFPPKPKDT LMISRTPEVT CVVVDVSHED PEVKFNWYVD        60
GVEVHNAKTK PREEQYNSTY RVVSVLTVLH QDWLNGKEYK CKVSNKALPS SIEKTISKAK        120
GQPREPQVYT LPPSRDELTK NQVSLTCLVK GFYPSDIAVE WESNGQPENN YKTTPPVLDS        180
DGSFFLYSKL TVDKSRWQQG NVFSCSVMHE ALHNHYTQKS LSLSPGK                     227
```

Further, the TACI-Fc fusion protein comprises the amino acid sequence as set forth in SEQ ID NO:6.

SEQ ID NO:6

```
EPKSSDKTHT CPPCPAPEAE GAPSVFLFPP KPKDTLMISR TPEVTCVVVD VSHEDPEVKF        60
NWYVDGVEVH NAKTKPREEQ YNSTYRVVSV LTVLHQDWLN GKEYKCKVSN KALPAPIEKT        120
ISKAKGQPRE PQVYTLPPSR DELTKNQVSL TCLVKGFYPS DIAVEWESNG QPENNYKTTP        180
PVLDSDGSFF LYSKLTVDKS RWQQGNVFSC SVMHEALHNH YTQKSLSLSP G                231
```

Further, the TACI-Fc fusion protein has an amino acid sequence with at least 75%, at least 80%, at least 85%, at least 90%, at least 95% or at least 99% identity to SEQ ID NO:7.

SEQ ID NO:7

```
SRVDQEERFP QGLWTGVAMR SCPEEQYWDP LLGTCMSCKT ICNHQSQRTC AAFCRSLSCR        60
KEQGKFYDHL LRDCISCASI CGQHPKQCAY FCENKLRSPV NLPPELDKTH TCPPCPAPEA        120
EGAPSVFLFP PKPKDTLMIS RTPEVTCVVV DVSHEDPEVK FNWYVDGVEV HNAKTKPREE        180
QYNSTYRVVS VLTVLHQDWL NGKEYKCKVS NKALPSSIEK TISKAKGQPR EPQVYTLPPS        240
RDELTKNQVS LTCLVKGFYP SDIAVEWESN GQPENNYKTT PPVLDSDGSF FLYSKLTVDK        300
SRWQQGNVFS CSVMHEALHN HYTQKSLSLS PGK                                    333
```

Further, the amino acid sequence of the TACI-Fc fusion protein is as set forth in SEQ ID NO:7.
Further, the TACI-Fc fusion protein has the amino acid sequence as set forth in SEQ ID NO:8.

SEQ ID NO:8

```
AMRSCPEEQY WDPLLGTCMS CKTICNHQSQ RTCAAFCRSL SCRKEQGKFY DHLLRDCISC        60
ASICGQHPKQ CAYFCENKLR SEPKSSDKTH TCPPCPAPEA EGAPSVFLFP PKPKDTLMIS        120
RTPEVTCVVV DVSHEDPEVK FNWYVDGVEV HNAKTKPREE QYNSTYRVVS VLTVLHQDWL        180
NGKEYKCKVS NKALPSSIEK TISKAKGQPR EPQVYTLPPS RDELTKNQVS LTCLVKGFYP        240
SDIAVEWESN GQPENNYKTT PPVLDSDGSF FLYSKLTVDK SRWQQGNVFS CSVMHEALHN        300
HYTQKSLSLS PGK                                                          313
```

Further, the TACI-Fc fusion protein has the amino acid sequence as set forth in SEQ ID NO:9.

SEQ ID NO:9

```
SLSCRKEQGE YYDHLLRDCI SCASICGQHP KQCADFCENK LRSGSGGGGS EPKSSDKTHT        60
CPPCPAPEAE GAPSVFLFPP KPKDTLMISR TPEVTCVVVD VSHEDPEVKF NWYVDGVEVH        120
NAKTKPREEQ YNSTYRVVSV LTVLHQDWLN GKEYKCKVSN KALPAPIEKT ISKAKGQPRE        180
PQVYTLPPSR DELTKNQVSL TCLVKGFYPS DIAVEWESNG QPENNYKTTP PVLDSDGSFF        240
LYSKLTVDKS RWQQGNVFSC SVMHEALHNH YTQKSLSLSP G                          281
```

EP 4 785 952 A1

Further, the drug targeting Blys and/or APRIL is telitacicept, atacicept, or povetacicept.

Further, the TACI-Fc fusion protein is telitacicept.

Further, the method comprises administering a therapeutically effective amount of the TACI-Fc fusion protein before solid organ transplantation.

Further, the method comprises administering a therapeutically effective amount of the TACI-Fc fusion protein after solid organ transplantation.

Further, the method comprises administering a therapeutically effective amount of the TACI-Fc fusion protein before and after solid organ transplantation.

Further, the patient comprises donor-specific antibodies (DSA) against human leukocyte antigen (HLA) before treatment.

Further, the method further comprises desensitization treatment before transplantation to remove or reduce donor-specific antibodies (DSA).

Further, the desensitization treatment comprises administering one or more of plasma exchange, immunoadsorption, intravenous immunoglobulin (IVIG), an anti-CD20 antibody, an anti-CD38 antibody, combined antibody removal (i.e. removal of antibodies already present or newly generated in the patient), a proteasome inhibitor, a complement inhibitor, a C1 esterase inhibitor (C1-INH), an IL-6/IL-6 receptor inhibitor, IgG-degrading enzyme of Streptococcus pyogenes (IdeS), a CTLA4-Fc fusion protein, a neonatal Fc receptor-targeting inhibitor, and a glucocorticoid for treatment.

Further, the anti-CD20 antibody includes rituximab, obinutuzumab, or the like; the anti-CD38 antibody includes daratumumab, felzartamab, or the like; the proteasome inhibitor includes bortezomib, carfilzomib, ixazomib, or the like; the complement inhibitor includes eculizumab or the like; the C1 esterase inhibitor includes Berinert and Cinryze; the IL-6/IL-6 receptor inhibitor includes tocilizumab, clazakizumab, or the like; the CTLA4-Fc fusion protein includes belatacept or the like; the neonatal Fc receptor-targeting inhibitor includes rozanolixizumab or the like.

Further, the solid organ includes, but is not limited to, one or more of a kidney, a heart, a liver, a lung, a pancreas, skin, a stomach and intestines.

Further, the antibody-mediated rejection of any one of the above includes but is not limited to antibody-mediated kidney transplant rejection, antibody-mediated liver transplant rejection, antibody-mediated heart transplant rejection, and antibody-mediated lung transplant rejection.

Further, the antibody-mediated rejection of any one of the above includes but is not limited to: one or more of active antibody-mediated rejection (Active ABMR), chronic active antibody-mediated rejection (Chronic active ABMR), chronic inactive antibody-mediated rejection (Chronic inactive ABMR), and C4d staining without evidence of rejection.

In some preferred embodiments, the antibody-mediated rejection is active antibody-mediated rejection (Active ABMR); in other preferred embodiments, the antibody-mediated rejection is chronic active antibody-mediated rejection (Chronic active ABMR); in other preferred embodiments, the antibody-mediated rejection is chronic inactive antibody-mediated rejection (Chronic inactive ABMR); in other preferred embodiments, the antibody-mediated rejection is C4d staining without evidence of rejection. It is understood that, clinically, antibody-mediated rejection may be classified into specific subtypes (that is, further determination as active antibody-mediated rejection (Active ABMR), chronic active antibody-mediated rejection (Chronic active ABMR), chronic inactive antibody-mediated rejection (Chronic inactive ABMR), C4d staining without evidence of rejection, or a combination of any two, three or four thereof), or may remain unclassified (uniformly referred to as antibody-mediated rejection); therefore, the specific disease subtype should not be construed as a limitation to the present invention.

Further, the method comprises administering to a patient having the antibody-mediated rejection a therapeutically effective amount of a drug targeting Blys and/or APRIL at a stage such as post-transplantation, a relapse stage, and after the failure of other therapeutic methods (the drug targeting Blys and/or APRIL is further preferably a TACI-Fc fusion protein, and still further preferably telitacicept).

Further, the patient is an adult patient or a pediatric patient. In some preferred embodiments, the patient is an adult patient. In some other preferred embodiments, the patient is a pediatric patient.

Further, the patient has not previously received a treatment regimen for antibody-mediated rejection.

Further, the patient has previously received a treatment regimen for antibody-mediated rejection.

Further, the treatment regimen for antibody-mediated rejection previously received by the patient comprises, but is not limited to, treatment with one or more of combined antibody removal (i.e., removal of antibodies already present or newly generated in the patient), a glucocorticoid, intravenous immunoglobulin, an anti-CD20 antibody, a proteasome inhibitor and/or complement blockade, and plasma exchange.

Further, the method comprises administering to a patient having the antibody-mediated rejection a therapeutically effective amount of the drug targeting Blys and/or APRIL, in combination with treatment by one or more methods among combined antibody removal (i.e., removal of antibodies already present or newly generated in the patient), glucocorticoids, intravenous immunoglobulin, anti-CD20 antibody, proteasome inhibitors and/or complement blockade, and plasma exchange.

Further, the single dose of the drug targeting Blys and/or APRIL is from about 0.1 to 10 mg/kg, further comprising 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10 mg/kg.

Further, the single dose of the drug targeting Blys and/or APRIL is 40-240 mg, further preferably 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg.

Further, the method for detecting the protein content of the above drug is: ultraviolet-visible spectrophotometry, wherein the absorbance value of the telitacicept sample at this wavelength is measured based on the fact that proteins have maximum ultraviolet absorption at 280 nm. After correcting for the absorbance at 320 nm, the absorbance value at 280 nm is proportional to the protein concentration, and the protein concentration is calculated according to the Lambert-Beer law to determine the protein content. The formula for calculating the protein content is as follows:

$$\text{Protein content (mg/ml)} = \frac{A280 \text{ (or A280 (corrected))}}{\varepsilon} * \text{Sample dilution factor}$$

wherein: $\varepsilon$ is the extinction coefficient of telitacicept, with a unit of $(\text{mg/ml})^{-1} \cdot \text{cm}^{-1}$;

$A_{280}$ is the average absorbance of the sample solution at 280 nm;

$A_{280}$ (corrected) is the average absorbance of the sample solution at 280 nm after correction.

Further, the drug targeting Blys and/or APRIL is used 1-8 times during a one-month interval, that is, the dosing frequency of the TACI-Fc fusion protein is 1, 2, 3, 4, 5, 6, 7, or 8 times per month.

Further, the drug targeting Blys and/or APRIL is used 1-8 times during a two-month interval, that is, the dosing frequency of the TACI-Fc fusion protein is 1, 2, 3, 4, 5, 6, 7, or 8 times per two months.

Further, the drug targeting Blys and/or APRIL is used 1-8 times during a three-month interval, that is, the dosing frequency of the TACI-Fc fusion protein is 1, 2, 3, 4, 5, 6, 7, or 8 times per three months.

Further, the dosing frequency of the drug targeting Blys and/or APRIL is once weekly, twice weekly or three times weekly.

Further, the dosing frequency of the drug targeting Blys and/or APRIL is once every two weeks or once every three weeks, once monthly.

Further, the dosing frequency of the drug targeting Blys and/or APRIL is as-needed dosing.

Further, the drug targeting Blys and/or APRIL is administered continuously and/or intermittently.

Further, the drug targeting Blys and/or APRIL is administered at regular intervals.

Further, the drug targeting Blys and/or APRIL is administered at irregular intervals.

Further, the administration mode of the drug targeting Blys and/or APRIL is subcutaneous, intramuscular or intravenous administration, and the administration site is preferably the thigh, the abdomen, or the upper arm. In some specific embodiments, the administration mode of the TACI-Fc fusion protein is subcutaneous injection, intramuscular injection or intravenous injection.

Further, the injection site of the drug targeting Blys and/or APRIL is the same or different for each injection. In some specific embodiments, the injection site of the TACI-Fc fusion protein is the same for each injection; in other specific embodiments, the injection site of the TACI-Fc fusion protein is different for each injection.

The present invention also provides a method for treating a patient with antibody-mediated rejection who has received a treatment regimen for antibody-mediated rejection, the method comprising (1) determining whether the patient has received a treatment regimen for antibody-mediated rejection, and (2) if the patient has previously received treatment for antibody-mediated rejection disease, administering to the patient with the antibody-mediated rejection an effective amount of a drug targeting Blys and/or APRIL, and further a TACI-Fc fusion protein.

The drug targeting Blys and/or APRIL (such as telitacicept) provided by the present invention exhibits unexpected clinical efficacy and good safety in the treatment of antibody-mediated rejection. Specifically, for antibody-mediated rejection (such as active antibody-mediated rejection (Active ABMR), chronic active antibody-mediated rejection (Chronic active ABMR), chronic inactive antibody-mediated rejection (Chronic inactive ABMR), C4d staining without evidence of rejection), the drug targeting Blys and/or APRIL (such as telitacicept) provided by the present invention exhibits good safety and better efficacy, and has excellent therapeutic potential. For patients with acute antibody-mediated rejection, the drug targeting Blys and/or APRIL provided by the present invention (such as telitacicept) all exhibited good safety and better efficacy; for patients with chronic active antibody-mediated rejection, the drug targeting Blys and/or APRIL provided by the present invention (such as telitacicept) all exhibited good safety and better efficacy; for chronic inactive antibody-mediated rejection and C4d staining without evidence of rejection, the drug targeting Blys and/or APRIL provided by the present invention (such as telitacicept) also exhibited good safety and better efficacy. For patients with antibody-mediated rejection who have failed treatment with conventional treatment regimens (such as plasma exchange, intravenous immunoglobulin, etc.) and antibodies (such as Darzalex, i.e. daratumumab), the use of the drug targeting Blys and/or APRIL provided by the present invention (such as telitacicept) can still exhibit good therapeutic effects. The drug targeting Blys and/or APRIL provided by the present invention (such as telitacicept) can exhibit better therapeutic effects in the treatment of patients with antibody-mediated rejection (especially chronic active antibody-mediated rejection) compared with conventional treatment regimens (such as plasma exchange, intravenous immunoglobulin, etc.) and antibodies (such as Darzalex, i.e. daratumumab). For patients with antibody-mediated rejection, the drug targeting Blys and/or APRIL provided by the present invention (such as telitacicept) in combination with other therapeutic means (such as conventional treatment regimens, glucocorticoids, plasma exchange, intravenous immunoglobulin, antibodies, etc.) can exhibit significant therapeutic effects. For patients with chronic active antibody-mediated rejection whose creatinine is maintained at a high level for a prolonged period, especially patients with chronic active antibody-mediated rejection whose creatinine shows no obvious downward trend after treatment, the drug targeting Blys and/or APRIL provided by the present invention (such as telitacicept) exhibits good therapeutic effects. For patients with antibody-mediated rejection who failed to respond to conventional treatment regimens (such as CellCept, tacrolimus capsules, prednisone tablets, and diltiazem tablets), the drug targeting Blys and/or APRIL (e.g., telitacicept) provided by the present invention can still exhibit good therapeutic effects.

### Brief Description of the Drawings

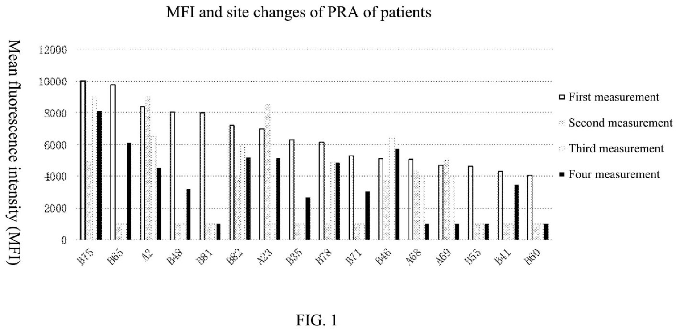

Figure 1 shows the changes in PRA-MFI and loci of patient Case 5 in Embodiment 1 (Note: in Figure 1, combination therapy with telitacicept was initiated after the first measurement).

### Detailed Description of Embodiments

Unless defined otherwise, all terms used herein have the same meanings as commonly understood by one of ordinary skill in the art. With regard to definitions and terminology in the art, professionals may specifically refer to Current Protocols in Molecular Biology (Ausubel).

The three-letter code and one-letter code for amino acids used in the present invention are as described in J. Biol. Chem., 243, p3558 (1968).

The term "TACI" in the present invention, i.e., transmembrane activator and CAML interactor, is a member of the tumor necrosis factor receptor superfamily. The term "BLys" in the present invention refers to B lymphocyte stimulator, which is a member of the TNF ligand superfamily that exists in two forms, membrane-bound and soluble, which is specifically expressed on the surface of bone marrow cells, and selectively stimulates B lymphocyte proliferation and immunoglobulin production. The term "APRIL" (a proliferation-inducing ligand) in the present invention is a tumor necrosis factor (TNF) analog that can stimulate the proliferation of naïve B cells and T cells in vivo and promote B cell accumulation. APRIL can specifically bind to TACI and BCMA, and after binding can prevent APRIL from binding to B cells and inhibit APRIL-stimulated naïve B cell proliferation response. Moreover, APRIL can compete with BLys for binding to receptors (BCMA, TACI).

The term "TACI-Fc fusion protein" involved in the present invention refers to transmembrane activator, calcium modulator and cyclophilin ligand interactor (TACI)-immunoglobulin fusion protein (i.e., TACI-Fc fusion protein), and the TACI-immunoglobulin fusion protein provided by the present invention comprises: (i) the TACI extracellular region or a fragment thereof that binds Blys and/or APRIL; and (ii) a human immunoglobulin constant region fragment.

The term "TACI extracellular region or a fragment thereof that binds Blys and/or APRIL" in the present invention includes the extracellular domain of TACI disclosed in U.S. Patent Nos. 5,969,102, 6,316,222 and 6,500,428 and U.S. Patent Applications 09/569,245 and 09/627,206 (the contents of which are incorporated herein by reference), as well as specific fragments of the TACI extracellular domain capable of interacting with TACI ligands, and the amino acid fragment at positions 13-118 of the TACI extracellular domain disclosed in Chinese Patent Publication No. CN101323643A.

Examples of "TACI-Fc fusion proteins" include telitacicept (amino acid sequence: SEQ ID NO:7), atacicept (amino acid sequence: SEQ ID NO:8), and povetacicept (amino acid sequence: SEQ ID NO:9).

In the term "human immunoglobulin constant region fragment" referred to in the present invention, the immunoglobulin portion is preferably IgG1, which may comprise a heavy chain constant region, such as a human heavy chain constant region. The preferred "human immunoglobulin constant region fragment" of the present invention is an amino acid fragment containing a partial hinge region domain, a CH2 domain, and a CH3 domain. In some more preferred embodiments, the amino acid sequence of the "human immunoglobulin constant region fragment" according to the present invention is as set forth in SEQ ID NO:4, or comprises an amino acid sequence having at least 90%, at least 91%, at least 92%, at least 93%, at least 94%, at least 95%, at least 96%, at least 97%, at least 98%, or at least 99% identity to SEQ ID NO:4. In some more preferred embodiments, the amino acid sequence of the "human immunoglobulin constant region fragment" is as set forth in SEQ ID NO:5.

The term "treatment" referred to in the present invention is associated with a given disease or condition, and includes but is not limited to: inhibiting the disease or condition, for example, arresting the development of the disease or condition; relieving the disease or condition, for example, causing regression of the disease or condition; or alleviating symptoms caused by the disease or condition, for example, alleviating, preventing or treating the symptoms of the disease or condition; or reducing the chance of disease recurrence or preventing disease recurrence.

The term "remission" as used in the present invention refers to the absence of clinical symptoms or signs associated with GPA, MPA or EGPA at the start or end of treatment.

The term "amino acid" as used in the present invention is understood in the broadest sense as a general term for a class of organic compounds containing an amino group and a carboxyl group, and preferably, the amino acids involved in the present invention are the main units that constitute proteins in living organisms, including but not limited to: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine and histidine.

The three-letter code and one-letter code for amino acids used in the present invention are as described in J. Biol. Chem., 243, p3558 (1968). There are multiple numbering schemes for amino acid positions, such as the Kabat numbering system, the EU numbering system, sequential numbering, and the like, and in the present invention, the amino acid positions are numbered using "sequential numbering", "positions 3, 8, 14, 15, 17, 110, 111 or 173 of SEQ ID NO:4" as described in the present invention refers to the amino acid at position 3, the amino acid at position 8 of SEQ ID NO:4, and so on; and "P3T" as described in the present invention refers to mutating the amino acid sequence at position 3 of SEQ ID NO:4 from the original "P" to "T", and "L8P" refers to mutating the amino acid sequence at position 8 of SEQ ID NO:4 from the original "L" to "P", and so on.

As an alternative embodiment, the constant region of the immunoglobulin provided by the present invention may introduce alterations of one or more amino acids, such as substitution (i.e. mutation), addition (i.e. insertion) or deletion.

The term "telitacicept" (or "Tai'ai", which are used interchangeably in the present invention) in the present invention is a TACI-Fc fusion protein, whose INN is telitacicept, the amino acid sequence of which is shown in SEQ ID NO:7, or as shown at https://extranet.who.int/soinn/mod/page/view.php?id=137&inn_n=10932.

The TACI-Fc fusion protein of the present invention can be administered by any of a variety of routes, including but not limited to: oral, intravenous injection, intramuscular injection, intraarterial injection, intramedullary injection, intraperitoneal injection, intrathecal injection, intracardiac, transdermal, percutaneous, topical, subcutaneous, intranasal, enteral, sublingual, intravaginal or rectal routes and the like.

The term "antibody-mediated rejection" in the present invention is also referred to as humoral rejection, and is an immunological injury resulting from a rejection reaction involving the participation of multiple humoral immune effector factors such as antibodies and complement. It plays an important pathogenic role in hyperacute rejection, acute rejection and chronic rejection.

The term "biological agent-based drug treatment regimen" in the present invention is commonly used for the treatment of patients with traditional treatment failure, glucocorticoid resistance or intolerance, relapse during glucocorticoid tapering, refractory or severe cases, and currently promising biological targeted therapies exemplarily include:

(1) B cell depletion therapy: such as, non-limitatively, anti-CD20 monoclonal antibody, anti-CD19 monoclonal antibody, B lymphocyte activating factor (BAFF) inhibitor and the like;
(2) targeting T lymphocytes: such as, non-limitatively, abatacept, signaling lymphocytic activation molecule family member 7 (SLAMF 7) monoclonal antibody, inducible co-stimulatory molecule ligand (ICOSL) inhibitor and the like;
(3) cytokine-targeting agents (IL-4, IL-5\TNF-$\alpha$) and intracellular signaling pathway-targeting JAK inhibitors, and the like.

The term "inactive/active" as used in the present invention refers to the presence of new, persistent or worsening clinical symptoms and/or signs associated with GPA, MPA or EGPA.

The term "severe" as used in the present invention refers to the presence of life-threatening symptoms or organ manifestations (such as alveolar hemorrhage, glomerulonephritis, central nervous system vasculitis and the like).

The term "about" in the present invention is used to indicate that a numerical value includes the inherent error variation of the device or method used to determine the numerical value, or the variation existing between samples being measured. Unless otherwise specified or evident from the context, the term "about" refers to within 10% above or below the reported numerical value (unless the number would exceed 100% of possible values or be below 0%). When used in conjunction with a numerical range or series, unless otherwise stated, the term "about" applies to the endpoints of the range or each numerical value listed in the series.

The implementations of the present invention will be described in detail below in conjunction with embodiments, but those skilled in the art will understand that the following embodiments are only used for illustrating the present invention and should not be regarded as limiting the scope of the present invention.

Embodiment 1 Therapeutic Effect of Real-World Cases

[Case 1]

The patient underwent deceased donor (DD) kidney transplantation, and received oral tacrolimus + mycophenolate mofetil + methylprednisolone triple immunosuppressive regimen for anti-rejection after surgery. Subsequently, due to elevated creatinine, the patient was given immunoadsorption and immunoglobulin treatment. Non-invasive donor-derived cell-free DNA detection was performed again, indicating a rejection risk coefficient of 0.46. Combined with other indicators, it was comprehensively considered as acute antibody-mediated rejection.
Subsequently, telitacicept treatment was performed, and the treatment regimen was as follows:

telitacicept 160 mg subcutaneously, once weekly, for a three-month course;
Subsequently, the telitacicept dose was adjusted to telitacicept 80 mg subcutaneously, once weekly, for a two-month course.

During telitacicept treatment, the patient's antibodies showed a slight rebound in the second month, but the antibodies continued to decline in the third month. During the use of telitacicept, the patient's renal function fluctuated between 120-150 umol/L, proteinuria remained persistently negative, there was no hepatotoxicity, and the patient had no obvious adverse reactions during injection. The results showed that for patients with acute antibody-mediated rejection, telitacicept exhibited good safety and therapeutic effects.

[Case 2]

After kidney transplantation, the patient received oral tacrolimus + mycophenolate mofetil for anti-rejection treatment. Subsequently, due to creatinine fluctuation and proteinuria, treatment with Tripterygium glycosides was administered but demonstrated poor efficacy. Combined with other indicators, it was comprehensively considered as chronic active antibody-mediated rejection.
Initially, the patient received PP (plasma exchange) + IVIG (intravenous immunoglobulin) combined with daratumumab treatment regimen: 6 courses of PP+IVIG treatment were given, and daratumumab 400 mg was administered by intravenous infusion after each of the last 5 treatments. Subsequently, due to a rebound in DR53 antibodies, the administration regimen was adjusted to intravenous infusion of tocilizumab once monthly, but the effects were all unsatisfactory.
Subsequently, the regimen was adjusted to subcutaneous injection of telitacicept once weekly, 80 mg each time.
At the follow-up visits in the first and third months of telitacicept use, the patient's DR53 antibodies and MFI values continued to decrease, renal function remained stable, proteinuria remained persistently negative, there was no hepatotoxicity, no obvious myelosuppression, no significant increase in infection risk, and the patient had no obvious adverse reactions during injection. The results showed:

① For patients with chronic active antibody-mediated rejection, telitacicept exhibited good safety and therapeutic effects.
② For patients with antibody-mediated rejection who have failed treatment with conventional treatment regimens (such as plasma exchange, intravenous immunoglobulin, etc.) and antibodies (such as Darzalex, i.e. daratumumab), the use of telitacicept can still exhibit good therapeutic effects.
③ Telitacicept is capable of exhibiting better therapeutic effect in the treatment of patients with antibody-mediated rejection (especially chronic active antibody-mediated rejection) as compared to conventional therapeutic regimens (such as plasma exchange, intravenous immunoglobulin, etc.), and antibodies (such as Darzalex, i.e., daratumumab).

[Case 3]

The patient underwent deceased donor (DD) kidney transplantation, followed by oral administration of tacrolimus + mycophenolate mofetil + methylprednisolone triple immunosuppressive regimen for anti-rejection therapy, and later, due to elevated creatinine, urine protein 3+, and in combination with other indicators, was comprehensively considered to have chronic active antibody-mediated rejection.
Subsequently, immunoadsorption therapy was performed, with the regimen as follows:

① rituximab 100 mg intravenous infusion;

② telitacicept 160 mg subcutaneously, once weekly, for a three-month course.

During treatment with telitacicept, the patient's urine protein was maintained at 2+, renal function remained relatively stable, without a significant increase in the risk of infection, and the patient had no obvious adverse reactions during the injection period. The results showed:

① For patients with chronic active antibody-mediated rejection, telitacicept exhibited good safety and therapeutic efficacy.

② For patients with antibody-mediated rejection, especially patients with chronic active antibody-mediated rejection, rituximab in combination with telitacicept can exhibit significant therapeutic efficacy.

[Case 4]

After the patient underwent allogeneic kidney transplantation, triple anti-rejection therapy was administered, and later, due to fatigue without obvious inducement, abnormal serum creatinine, and proteinuria, and in combination with other indicators, was comprehensively considered to have chronic active antibody-mediated rejection.

Subsequently, CellCept (4 tablets, twice daily), tacrolimus capsules (1 mg specification, 5 capsules, twice daily), prednisone tablets (5 mg specification, 5 tablets, once daily), and diltiazem tablets (30 mg specification, 2 tablets, three times daily) conventional therapy was performed, but the creatinine decrease was not obvious.

The treatment regimen was adjusted to add telitacicept on the basis of the above conventional treatment regimen, with the regimen as follows:

① telitacicept 160 mg, administered subcutaneously, once a week, for a total course of three months.

② Subsequently, the dose of telitacicept was adjusted to 80 mg, subcutaneous administration, once a week, for a total course of three months.

The patient's creatinine was maintained at a high creatinine level (190-200) for a long time, and there was no obvious downward trend after conventional treatment. However, one month after the use of telitacicept, there was a significant decrease, and during the administration period, creatinine showed an overall good downward trend (decreased to 162), and during this period, renal function remained stable, proteinuria remained stable, and the patient had no obvious adverse reactions during the injection period. The results showed:

① For patients with chronic active antibody-mediated rejection, telitacicept exhibited good safety and therapeutic efficacy.

② For patients with chronic active antibody-mediated rejection whose creatinine is maintained at a high level for a prolonged period, especially for patients whose creatinine shows no obvious downward trend after treatment, telitacicept exhibits good therapeutic efficacy.

③ For patients with antibody-mediated rejection, especially for patients with chronic active antibody-mediated rejection, telitacicept can exhibit superior therapeutic effects compared with conventional therapeutic means (such as CellCept, tacrolimus capsules, prednisone tablets, and diltiazem tablets).

④ For patients with antibody-mediated rejection who have failed treatment with conventional therapeutic regimens (such as CellCept, tacrolimus capsules, prednisone tablets, and diltiazem tablets), the use of telitacicept can still exhibit good therapeutic effects.

⑤ Telitacicept in combination with traditional therapeutic regimens (such as CellCept, tacrolimus capsules, prednisone tablets, and diltiazem tablets) for the treatment of patients with antibody-mediated rejection (especially patients with chronic active antibody-mediated rejection) can exhibit good therapeutic effects.

[Case 5]

The patient had proteinuria for 5 years and hemodialysis for more than 2 years, and for preoperative evaluation of kidney transplantation, PRA examination showed Class I positive and Class II negative. HLA-specific antibody detection results: multiple loci were positive, and in combination with other indicators, the patient was comprehensively considered as a highly sensitized kidney transplant recipient.

Conventional treatment in combination with telitacicept treatment, 160 mg/week, subcutaneous injection.

After 4 months of treatment with the above regimen, the patient's urine microalbumin was improved, HLA antibodies were reduced, and compared with the first measurement, after treatment with telitacicept, the MFI values of DSA in the fourth measurement showed an overall downward trend, and the number of DSA-positive loci was reduced.

The MFI and locus changes of PRA in the patient are shown in Figure 1 (note: in Figure 1, telitacicept was used for combination treatment after the first detection).

The treatment results showed that telitacicept can reduce HLA antibodies, reduce the number of DSA loci and DSA intensity, and effectively improve urine microalbumin, and has good effect in delaying the progression of pathological damage of antibody-mediated rejection of the transplanted kidney and improving the long-term prognosis of transplanted kidney.

In summary, the application of telitacicept in patients with antibody-mediated rejection exhibited better safety and superior therapeutic effects compared with existing therapeutic regimens/modalities. Specifically, for antibody-mediated rejection (such as active antibody-mediated rejection (Active ABMR), chronic active antibody-mediated rejection (Chronic active ABMR), chronic inactive antibody-mediated rejection (Chronic inactive ABMR), and C4d staining without evidence of rejection), telitacicept exhibited good safety and better efficacy, and has excellent therapeutic potential. For patients with acute antibody-mediated rejection, telitacicept exhibited good safety and better efficacy; for patients with chronic active antibody-mediated rejection, telitacicept exhibited good safety and better efficacy; for chronic inactive antibody-mediated rejection and C4d staining without evidence of rejection, telitacicept also exhibited good safety and better efficacy. For patients with antibody-mediated rejection who have failed treatment with traditional therapeutic regimens (such as plasma exchange, intravenous immunoglobulin, etc.) and antibodies (such as Darzalex, i.e., daratumumab), the use of telitacicept can still exhibit good therapeutic effects. For highly sensitized kidney transplant recipients, treatment with telitacicept exhibits good therapeutic effect. Telitacicept is capable of exhibiting better therapeutic effect in the treatment of patients with antibody-mediated rejection (especially chronic active antibody-mediated rejection) as compared to conventional therapeutic regimens (such as plasma exchange, intravenous immunoglobulin, etc.), and antibodies (such as Darzalex, i.e., daratumumab). For patients with antibody-mediated rejection, telitacicept in combination with other therapeutic means (such as conventional therapeutic regimens, glucocorticoids, plasma exchange, intravenous immunoglobulin, antibodies, and the like) is capable of demonstrating significant therapeutic effect. For patients with chronic active antibody-mediated rejection whose creatinine is maintained at a high level for a prolonged period, especially patients with chronic active antibody-mediated rejection whose creatinine shows no obvious downward trend after treatment, telitacicept exhibits good therapeutic effect. For patients with antibody-mediated rejection who have failed treatment with conventional therapeutic regimens (such as CellCept, tacrolimus capsules, prednisone tablets, diltiazem tablets), use of telitacicept can still exhibit good therapeutic effect. For the treatment of highly sensitized kidney transplant recipients, conventional treatment combined with telitacicept treatment exhibits good therapeutic effect.

Embodiment 2 Clinical Trial Study

Primary Objective

1. To evaluate the efficacy of telitacicept in treating or alleviating antibody-mediated rejection (such as chronic active antibody-mediated rejection and/or acute antibody-mediated rejection).

2. Secondary Objective: To preliminarily evaluate the safety of telitacicept in treating refractory and/or alleviating antibody-mediated rejection (such as chronic active antibody-mediated rejection and/or acute antibody-mediated rejection).

The above description is only of preferred embodiments, which serve as examples only and do not limit the combination of features necessary for carrying out the present invention. The headings provided are not intended to limit the various embodiments of the present invention. Terms such as "comprising", "containing" and "including" are not intended to be limiting. Furthermore, unless otherwise stated, the singular includes the plural when not modified by a numeral modifier, and "or" means "and/or". Unless defined otherwise herein, all technical and scientific terms used herein have the same meanings as commonly understood by one of ordinary skill in the art.

All publications and patents mentioned in this application are incorporated herein by reference. Various modifications and variations of the described methods and compositions of the present invention will be apparent to one of ordinary skill in the art without departing from the scope and spirit of the present invention. Although the present invention has been described by way of specific preferred implementations, it should be understood that the invention as claimed should not be unduly limited to these specific implementations. In fact, various modifications of the described modes for carrying out the present invention that are apparent to one of ordinary skill in the relevant art are intended to be within the scope of the appended claims.

**Claims**

1. A method for preventing, treating, or alleviating antibody-mediated rejection, the method comprising administering to a patient having the antibody-mediated rejection a therapeutically effective amount of a drug targeting Blys and/or APRIL.

2. The method of claim 1, wherein the patient is a patient who is to receive, is receiving or has received solid organ transplantation.

3. The method of claim 1 or 2, wherein the drug targeting Blys and/or APRIL is a TACI-Fc fusion protein.

4. The method of claim 3, wherein the TACI-Fc fusion protein comprises:

    (i) a TACI extracellular region or a fragment thereof that binds Blys and/or APRIL; and
    (ii) a human immunoglobulin constant region fragment.

5. The method of claim 4, wherein the TACI extracellular region or a fragment thereof that binds Blys and/or APRIL comprises the amino acid sequence set forth in SEQ ID NO:1 or SEQ ID NO:2 or SEQ ID NO:3.

6. The method of claim 4, wherein the human immunoglobulin is IgG1, or the human immunoglobulin constant region fragment comprises the amino acid sequence set forth in SEQ ID NO:4 or comprises an amino acid sequence having at least 90%, at least 91%, at least 92%, at least 93%, at least 94%, at least 95%, at least 96%, at least 97%, at least 98%, or at least 99% identity to SEQ ID NO:4.

7. The method of claim 6, wherein the human immunoglobulin constant region fragment comprises a modification of an amino acid on one or more positions corresponding to positions 3, 8, 14, 15, 17, 110, 111 or 173 of SEQ ID NO:4.

8. The method of claim 7, wherein the human immunoglobulin constant region fragment comprises amino acid modifications at 1, 2, 3, 4, 5, 6, 7, 8 or more positions as compared to SEQ ID NO:4.

9. The method of claim 7 or 8, wherein the modification is a substitution, deletion, or insertion of amino acids.

10. The method of claim 9, wherein the substitution comprises one or more of P3T, L8P, L14A, L15E, G17A, A110S, P111S and A173T.

11. The method of claim 9, wherein the insertion is the insertion of 1, 2, 3, 4, 5, 6, 7, 8 or more amino acids at the N-terminus of the human immunoglobulin constant region fragment.

12. The method of claim 7 or 8, wherein the human immunoglobulin constant region fragment comprises the amino acid sequence of SEQ ID NO:5 or SEQ ID NO:6.

13. The method of claim 12, wherein the TACI-Fc fusion protein has an amino acid sequence with at least 75%, at least 80%, at least 85%, at least 90%, at least 95% or at least 99% identity to SEQ ID NO:7.

14. The method of claim 12 or 13, wherein the TACI-Fc fusion protein has the amino acid sequence set forth in SEQ ID NO:7 or SEQ ID NO:8 or SEQ ID NO:9.

15. The method of claim 14, wherein the TACI-Fc fusion protein is telitacicept, atacicept, or povetacicept.

16. The method of any one of claims 2 to 3, wherein a therapeutically effective amount of the TACI-Fc fusion protein is administered before solid organ transplantation.

17. The method of any one of claims 2 to 3, wherein a therapeutically effective amount of the TACI-Fc fusion protein is administered after solid organ transplantation.

18. The method of any one of claims 2 to 3, wherein a therapeutically effective amount of the TACI-Fc fusion protein is administered before and after solid organ transplantation.

19. The method of any one of claims 1 to 3 or 16, wherein the patient comprises donor-specific antibodies (DSA) against human leukocyte antigen (HLA) before treatment.

20. The method of claim 19, wherein the method further comprises desensitization treatment before transplantation to remove or reduce donor-specific antibodies (DSA).

21. The method of claim 20, wherein the desensitization treatment comprises administering one or more of plasma exchange, immunoadsorption, intravenous immunoglobulin (IVIG), an anti-CD20 antibody, an anti-CD38 antibody, combined antibody removal (i.e., removal of antibodies already present or newly generated in the patient), a proteasome inhibitor, a complement inhibitor, a C1 esterase inhibitor (C1-INH), an IL-6/IL-6 receptor inhibitor, IgG-degrading enzyme of Streptococcus pyogenes (IdeS), a CTLA4-Fc fusion protein, a neonatal Fc receptor-targeting inhibitor, and a glucocorticoid for treatment.

22. The method of any one of claims 1 to 21, wherein the solid organ comprises, but is not limited to, one or more of a kidney, a heart, a liver, a lung, a pancreas, skin, a stomach, and intestines.

23. The method of any one of claims 1 to 22, wherein the antibody-mediated rejection comprises, but is not limited to, one or more of antibody-mediated kidney transplant rejection, antibody-mediated liver transplant rejection, antibody-mediated heart transplant rejection, and antibody-mediated lung transplant rejection.

24. The method of any one of claims 1 to 23, wherein the antibody-mediated rejection comprises, but is not limited to, one or more of active antibody-mediated rejection (Active ABMR), chronic active antibody-mediated rejection (Chronic active ABMR), chronic inactive antibody-mediated rejection (Chronic inactive ABMR), and C4d staining without evidence of rejection.

25. The method of any one of claims 1 to 24, wherein the method comprises administering a therapeutically effective amount of a drug targeting Blys and/or APRIL to a patient having the antibody-mediated rejection at a stage such as post-transplantation, a relapse stage, or after failure of other treatment methods.

26. The method of any one of claims 1 to 25, wherein the patient is an adult patient or a pediatric patient.

27. The method of any one of claims 1 to 26, wherein the patient has not previously received a treatment regimen for antibody-mediated rejection.

28. The method of any one of claims 1 to 27, wherein the patient has previously received a treatment regimen for antibody-mediated rejection.

29. The method of any one of claims 28, wherein the treatment regimen for antibody-mediated rejection previously received by the patient comprises, but is not limited to, treatment with one or more of combined antibody removal (i.e., removal of antibodies already present or newly generated in the patient), a glucocorticoid, intravenous immunoglobulin, an anti-CD20 antibody, a proteasome inhibitor and/or complement blockade, and plasma exchange.

30. The method of any one of claims 1 to 29, wherein the method comprises administering a therapeutically effective amount of the drug targeting Blys and/or APRIL to the patient having the antibody-mediated rejection in combination with one or more of a conventional treatment, antibody removal (i.e., removal of antibodies already present or newly generated in the patient), a glucocorticoid, intravenous immunoglobulin, an anti-CD20 antibody, a proteasome inhibitor and/or complement blockade, and plasma exchange for treatment; preferably, the anti-CD20 antibody is rituximab.

31. The method of any one of claims 1 to 30, wherein a single dose of the drug targeting Blys and/or APRIL is about 0.1 to 10 mg/kg.

32. The method of any one of claims 1 to 31, wherein a single dose of the drug targeting Blys and/or APRIL is 40-240 mg; further preferably 40 mg, 60 mg, 80 mg, 100 mg, 120 mg, 140 mg, 160 mg, or 240 mg; still further preferably 80 mg or 160 mg.

33. The method of any one of claims 1 to 32, wherein a dosing frequency of the drug targeting Blys and/or APRIL is as-needed dosing.

34. The method of any one of claims 1 to 33, wherein the drug targeting Blys and/or APRIL is administered 1-8 times per month, and/or 1-8 times per two-month interval, and/or 1-8 times per three-month interval, or the dosing frequency is once weekly, or once every two weeks, or once every three weeks, or once monthly.

35. The method of any one of claims 1 to 34, wherein an administration mode of the drug targeting Blys and/or APRIL is subcutaneous, intramuscular, or intravenous administration, or an administration site is the thigh, the abdomen, or the upper arm.

MFI and site changes of PRA of patients

FIG. 1

## INTERNATIONAL SEARCH REPORT

| International application No. |
| --- |
| **PCT/CN2024/121600** |

| A. | CLASSIFICATION OF SUBJECT MATTER |
| --- | --- |

A61K 39/395(2006.01)i; A61K38/17(2006.01)i; A61P37/06(2006.01)i

According to International Patent Classification (IPC) or to both national classification and IPC

| B. | FIELDS SEARCHED |
| --- | --- |

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, EBI, ISI Web of Science, CNKI, BAIDU, BING: TACI, TACI-Fc, Blys, APRIL, 抗体介导性排斥反应, 移植, 排斥, Antibody-mediated rejection, ABMR, AMR, 体液性排斥反应, Humoral rejection, graft, transplantation, rejection, Telitacicept, 泰它西普, Atacicept, Povetacicept

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2011109280 A1 (LERNER RESEARCH INSTITUTE et al.) 09 September 2011 (2011-09-09) description, page 8, paragraph 2, page 11, paragraphs 1-2, page 29, last paragraph, page 32, last paragraph, and page 39, paragraph 1 | 1-35 |
| X | US 2007274984 A1 (ARES TRADING S.A. et al.) 29 November 2007 (2007-11-29) description, paragraph 16 | 1-35 |
| A | CN 113573732 A (REMEGEN BIOPHARMACEUTICAL (YANTAI) CO., LTD.) 29 October 2021 (2021-10-29) entire document | 1-35 |
| A | CN 116867507 A (REMEGEN BIOPHARMACEUTICAL (YANTAI) CO., LTD.) 10 October 2023 (2023-10-10) entire document | 1-35 |
| A | CN 105770891 A (ZYMOGENETICS INC. et al.) 20 July 2016 (2016-07-20) entire document | 1-5 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 December 2024** | **25 December 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/121600**

| C. | DOCUMENTS CONSIDERED TO BE RELEVANT | |
|---|---|---|
| Category* | Citation of document, with indication, where appropriate, of the relevant passages | Relevant to claim No. |
| A | CN 116568327 A (CSL BEHRING GMBH) 08 August 2023 (2023-08-08)<br>entire document | 1-35 |

Form PCT/ISA/210 (second sheet) (July 2022)

**INTERNATIONAL SEARCH REPORT**

International application No.

**PCT/CN2024/121600**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/121600**

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **1-35**
because they relate to subject matter not required to be searched by this Authority, namely:

Claims 1-35 relate to a method for preventing, treating or alleviating antibody-mediated rejection, which falls within methods for treatment of diseases as defined in PCT Rule 39.1(iv). The present report is provided on the basis of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/121600**

| Patent document cited in search report | | | Publication date (day/month/year) | Patent family member(s) | | | Publication date (day/month/year) |
|---|---|---|---|---|---|---|---|
| WO | 2011109280 | A1 | 09 September 2011 | None | | | |
| US | 2007274984 | A1 | 29 November 2007 | JP | 2009537563 | A | 29 October 2009 |
| | | | | CA | 2651791 | A1 | 22 November 2007 |
| | | | | ZA | 200809202 | B | 30 December 2009 |
| | | | | WO | 2007134326 | A2 | 22 November 2007 |
| | | | | WO | 2007134326 | A3 | 17 April 2008 |
| | | | | AR | 060935 | A1 | 23 July 2008 |
| | | | | US | 8784812 | B2 | 22 July 2014 |
| | | | | KR | 20090016707 | A | 17 February 2009 |
| | | | | AU | 2007249223 | A1 | 22 November 2007 |
| | | | | AU | 2007249223 | B2 | 02 August 2012 |
| | | | | IL | 195243 | A0 | 01 August 2011 |
| | | | | EP | 2035028 | A2 | 18 March 2009 |
| | | | | MX | 2008014365 | A | 27 November 2008 |
| | | | | JP | 2013100313 | A | 23 May 2013 |
| | | | | NZ | 572373 | A | 24 February 2012 |
| | | | | EA | 200870535 | A1 | 28 April 2009 |
| | | | | EA | 015342 | B1 | 30 June 2011 |
| | | | | UA | 98462 | C2 | 25 May 2012 |
| | | | | BRPI | 0711823 | A2 | 17 January 2012 |
| | | | | ECSP | 088982 | A | 30 January 2009 |
| CN | 113573732 | A | 29 October 2021 | None | | | |
| CN | 116867507 | A | 10 October 2023 | WO | 2023016444 | A1 | 16 February 2023 |
| | | | | EP | 4292603 | A1 | 20 December 2023 |
| | | | | US | 2024002468 | A1 | 04 January 2024 |
| | | | | BR | 112023018745 | A2 | 12 March 2024 |
| | | | | AU | 2022325359 | A1 | 18 May 2023 |
| | | | | AU | 2022325359 | A9 | 29 June 2023 |
| | | | | KR | 20240005815 | A | 12 January 2024 |
| | | | | JP | 2024510636 | A | 08 March 2024 |
| | | | | TW | 202333770 | A | 01 September 2023 |
| | | | | CA | 3196569 | A1 | 16 February 2023 |
| CN | 105770891 | A | 20 July 2016 | SG | 182191 | A1 | 30 July 2012 |
| | | | | JP | 2010530000 | A | 02 September 2010 |
| | | | | PT | 2167038 | T | 01 June 2018 |
| | | | | LT | 2167038 | T | 25 May 2018 |
| | | | | IL | 202305 | A0 | 30 June 2010 |
| | | | | IL | 202305 | B | 28 June 2018 |
| | | | | SI | 2167038 | T1 | 31 August 2018 |
| | | | | JP | 2015013887 | A | 22 January 2015 |
| | | | | HUE | 038445 | T2 | 29 October 2018 |
| | | | | CY | 1120830 | T1 | 11 December 2019 |
| | | | | DK | 2167038 | T3 | 06 August 2018 |
| | | | | PL | 2167038 | T3 | 31 October 2018 |
| | | | | ES | 2681195 | T3 | 12 September 2018 |
| | | | | EA | 200901630 | A1 | 30 June 2010 |
| | | | | EA | 022911 | B1 | 31 March 2016 |
| | | | | EP | 2167038 | A2 | 31 March 2010 |
| | | | | EP | 2167038 | B1 | 25 April 2018 |
| | | | | CA | 2690119 | A1 | 24 December 2008 |

Form PCT/ISA/210 (patent family annex) (July 2022)

**INTERNATIONAL SEARCH REPORT**
Information on patent family members

International application No.

**PCT/CN2024/121600**

| Patent document cited in search report | | | Publication date (day/month/year) | Patent family member(s) | | | Publication date (day/month/year) |
|---|---|---|---|---|---|---|---|
| | | | | TR | 201806960 | T4 | 21 June 2018 |
| | | | | US | 2009186040 | A1 | 23 July 2009 |
| | | | | AU | 2008265974 | A1 | 24 December 2008 |
| | | | | AU | 2008265974 | A8 | 07 January 2010 |
| | | | | AU | 2008265974 | B2 | 29 August 2013 |
| | | | | HRP | 20181184 | T1 | 21 September 2018 |
| | | | | WO | 2008157369 | A2 | 24 December 2008 |
| | | | | WO | 2008157369 | A3 | 19 February 2009 |
| | | | | MX | 2009013183 | A | 20 January 2010 |
| | | | | JP | 2017031209 | A | 09 February 2017 |
| | | | | ZA | 200908274 | B | 29 February 2012 |
| CN | 116568327 | A | 08 August 2023 | CA | 3198740 | A1 | 27 May 2022 |
| | | | | JP | 2023551193 | A | 07 December 2023 |
| | | | | WO | 2022109124 | A1 | 27 May 2022 |
| | | | | US | 2024000908 | A1 | 04 January 2024 |
| | | | | AU | 2021381360 | A1 | 22 June 2023 |
| | | | | EP | 4247416 | A1 | 27 September 2023 |
| | | | | KR | 20230110563 | A | 24 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5969102 A
- US 6316222 B
- US 6500428 B
- US 569245
- US 09627206 B
- CN 101323643 A

### Non-patent literature cited in the description

- **SELLARÉS J** ; **DE FREITAS DG** ; **MENGEL M et al.** Understanding the causes of kidney transplant failure: the dominant role of antibody-mediated rejection and nonadherence. *Am J Transplant*, 2012, vol. 12, 388-399
- **LOUPY A et al.** The Banff 2019 Kidney Meeting Report (I): Updates on and clarification of criteria for T cell- and antibody-mediated rejection. *Am J Transplant.*, September 2020, vol. 20 (9), 2318-2331
- **RODRIGUEZ-RAMIREZ S** ; **AL JURDI A** ; **KONVALINKA A** ; **RIELLA LV**. Antibody-mediated rejection: prevention, monitoring and treatment dilemmas. *Curr Opin Organ Transplant*, 01 October 2022, vol. 27 (5), 405-414
- **BONOMINI V** ; **VANGELISTA A** ; **FRASCÀ GM et al.** Effects of plasmapheresis in renal transplant rejection. A controlled study. *Trans Am Soc Artif Intern Organs*, 1985, vol. 31, 698-703
- **ALLEN NH** ; **DYER P** ; **GEOGHEGAN T**. Plasma exchange in acute renal allograft rejection. A controlled trial. *Transplantation*, 1983, vol. 35, 425-428
- **SAUTENET B** ; **BLANCHO G** ; **BÜCHLER M et al.** One-year results of the effects of rituximab on acute antibody-mediated rejection in renal transplantation: RITUX ERAH, a multicenter double-blind randomized placebo-controlled trial. *Transplantation*, 2016, vol. 100, 391-399
- **LAWS LH** ; **PARKER CE** ; **CHERALA G et al.** Inflammation causes resistance to anti-CD20-mediated B cell depletion. *Am J Transplant*, 2016, vol. 16, 3139-3149
- **JORDAN SC** ; **TYAN D** ; **STABLEIN D et al.** Evaluation of intravenous immunoglobulin as an agent to lower allosensitization and improve transplantation in highly sensitized adult patients with end-stage renal disease: report of the NIH IG02 trial[J. *J Am Soc Nephrol*, 2004, vol. 15 (12), 3256-3262
- **VIEIRA CA** ; **AGARWAL A** ; **BOOK BK et al.** Rituximab for reduction of anti-HLA antibodies in patients awaiting renal transplantation: 1. Safety, pharmacodynamics, and pharmacokinetics[J. *Transplantation*, 2004, vol. 77 (4), 542-548
- **WANG ZHIYONG** ; **ZHANG GENG** ; **YUAN JIANLIN**. Research progress in preoperative desensitization treatment for pre-sensitized patients with kidney disease before transplantation [J. *Chinese Journal of Organ Transplantation*, 2017, vol. 38 (12), 761-764
- *J. Biol. Chem.*, 1968, vol. 243, 3558